(19) Europäisches Patentamt
European Patent Office
Office européen des brevets

(11) Veröffentlichungsnummer: **0 305 774**
**B1**

(12) # EUROPÄISCHE PATENTSCHRIFT

(45) Veröffentlichungstag der Patentschrift: **01.08.90**

(51) Int. Cl.⁵: **B01J 31/10**, C07C 37/20

(21) Anmeldenummer: **88112921.7**

(22) Anmeldetag: **09.08.88**

(54) **Mit Thiazolinen modifizierte Ionenaustauscher.**

(30) Priorität: **19.08.87 DE 3727641**

(43) Veröffentlichungstag der Anmeldung:
**08.03.89 Patentblatt 89/10**

(45) Bekanntmachung des Hinweises auf die Patenterteilung:
**01.08.90 Patentblatt 90/31**

(84) Benannte Vertragsstaaten:
**BE DE FR GB IT NL**

(56) Entgegenhaltungen:
**EP-A- 0 023 325**
**DE-A- 2 422 532**
**GB-A- 1 183 564**
**US-A- 4 396 728**

(73) Patentinhaber: **BAYER AG,**
**D-5090 Leverkusen 1 Bayerwerk(DE)**

(72) Erfinder: **Rudolph, Udo, Dr., Scheiblerstrasse 101,**
**D-4150 Krefeld(DE)**
Erfinder: **Wulff, Claus, Dr., Richard-Strauss-Strasse 21,**
**D-4150 Krefeld(DE)**
Erfinder: **Hinz, Jürgen, Dr., Kemmerhofstrasse 181,**
**D-4150 Krefeld(DE)**
Erfinder: **Bachem, Norbert, Dr., Jentgesallee 43,**
**D-4150 Krefeld(DE)**

**EP 0 305 774 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen mit Thiazolinen modifizierten Ionenaustauscher und seine Verwendung bei der Herstellung von Kondensationsprodukten, vorzugsweise Bisphenolen.

Die Kondensation von Phenolen und Carbonylverbindungen zur Bildung von Bisphenolen ist bekannt. Bei dieser Reaktion wurden bereits die verschiedensten Katalysatoren verwendet, beispielsweise Chlorwasserstoffsäure (US-PS 2 182 308 und 2 191 831), Bortrifluorid (Chemical Abstracts 58, 3338 c), Perchlorsäure (Chemical Abstracts 60, 1626 h), Benzolsulfonsäure (Chemical Abstracts 59, 511 h) und die verschiedensten Kationenaustauschharze (z. B. GB-PS 842 209, 849 565 und 883 391). Auch der Zusatz von S-haltigen Verbindungen zum Katalysator ist bekannt, z. B. US-PS 2 468 982, 2 623 908 die Verwendung von Thioglykolsäure und 3-Merkaptopropionsäure, aus der US-PS 2 359 242 der Zusatz von Thiophenolen, aus der US-PS 2 775 620 der Zusatz von Alkylmercaptanen, aus Chemical Abstracts 58, 1403 e der Zusatz von Schwefelwasserstoff.

Die bekannten, S-haltigen Katalysatoren können in der betrieblichen Praxis zu erheblichen Korrosionsschäden führen. Die mit diesen Katalysatoren hergestellten Bisphenole sind verunreinigt. Die Rohprodukte enthalten neben Bisphenol nicht umgesetztes Phenol, Carbonylverbindung, Reaktionswasser und noch unerwünschte Nebenprodukte, z. B. bei der Bisphenol A Synthese Isomere des Bisphenols A, insbesondere 2.2-(2.4'Dihydroxidiphenyl)-propan und 2.2(2.2'-Dihydroxidiphenyl)-propan, komplexe Produkte, wie das sogenannte "Codimere" 2.2.4-Trimethyl-4-p-hydroxiphenylchroman, Kondensationsprodukte, wie Trisphenol oder noch höher kondensierte Produkte in Form teeriger, auch hochsiedender Substanzen. Die Anwesenheit dieser Nebenprodukte ist unerwünscht, da sie dazu neigen, im Endprodukt zu verbleiben und Verfärbungen hervorzurufen. Weiterhin bereiten die Nebenprodukte unter Umständen erhebliche Schwierigkeiten bei der Weiterverarbeitung der Produkte.

Darüber hinaus verhindern die anwesenden Nebenprodukte einige der üblichen Reaktionen des Bisphenols, insbesondere die weitere Umsetzung zu Polycarbonaten.

Die US-PS 3 394 089 beschreibt ein Verfahren zur Herstellung von Bisphenol A aus Aceton und Phenol unter Verwendung eines sulfonsäuregruppenhaltigen Katalysators, dessen Sulfonsäuregruppen zu 5 bis 25 mol % mit Mercaptoaminen unter Ammonsalzbildung blockiert sind, das diese Nachteile vermeiden soll. Dieses modifizierte Ionenaustauscherharz, das durch Neutralisation mit z. B. β-Mercaptoethylamin in wäßriger Lösung erhalten wird, bereitet bei Anwendung im großtechnischen Maßstab Probleme, weil es instabil ist und die Mercaptoverbindung im Dauerversuch durch das Reaktionsmedium ausgewaschen wird.

Es wurde nun gefunden, daß stark saure Ionenaustauscher die einen sehr hohen bis quantitativen Neutralisationsgrad der Säuregruppen aufweisen und die Neutralisation mit vorher getrocknetem Ionenaustauscher in wasserfreiem Medium vorgenommen wird, sich z. B. zur Herstellung von hochreinen Bisphenolen gut eignen.

Gegenstand der Erfindung ist daher ein mit Thiazolinen modifizierter Ionenaustauscher, der dadurch gekennzeichnet ist, daß man bekannte saure Ionenaustauscherharze, mit Totalkapazitäten an Säurefunktionen in wasserfeuchter Form mit einem Wassergehalt von ca. 75 bis 85 Gew.-%, von 0,7 bis 2,1 mval/ml Ionenaustauscher oder mit Totalkapazitäten an Säurefunktionen von 3,5 bis 5 mval, bezogen auf 1 g Trockensubstanz, an Ionenaustauscher, trocknet, danach mit dem für die Bisphenolherstellung einzusetzenden Phenol spült und danach oberhalb des Schmelzpunktes dieses Phenols mit mindestens 0,3 Molen, vorzugsweise mit 0,4 Molen bis 1 Mol, pro Mol Säurefunktion der Ionenaustauscher, an Thiazolinen der Formel (I) neutralisiert.

Die Säurefunktion dieser modifizierten Ionenaustauscher sind neutralisiert zu mindestens 10 Mol-%, vorzugsweise von 20 Mol-% bis 100 Mol-% mit dem Thiazolin der Formel (I)

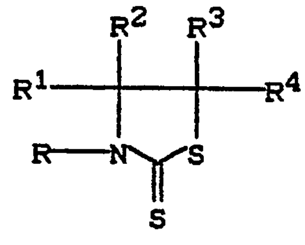

(I),

in welcher

die Reste R, $R^1$, $R^2$, $R^3$, $R^4$ unabhängig voneinander für Wasserstoff, einen $C_1$ - $C_6$-Alkyl-, einen $C_5$ - $C_{10}$ Cycloalkyl- oder einen $C_6$ - $C_{14}$-Arylrest stehen.

Geeignete saure Ionenaustauscher sind z. B. (handels)-übliche Umsetzungsprodukte von Styrol-Divinylbenzol-Copolymerisaten mit üblichen Sulfonierungsmitteln, wie Schwefelsäure, Chlorsulfonsäure u. a. Die Ionenaustauscher können in Kugelform vorliegen und Korngrößen von 0,3 - 1,5 mm Durchmesser aufweisen. Sie können vom Geltyp oder makroporös sein. Ihre Totalkapazität an Säurefunktionen in wasserfeuchter Form mit einem Wassergehalt von ca. 75 bis 85 Gew.-% reicht von 0,7 bis 2,1 mval/ml Ionenaustauscher oder von 3,5 bis 5 mval, bezogen auf 1 g Trockensubstanz an Ionenaustauscher.

Diese Ionenaustauscher werden gegebenenfalls durch Wärme, gegebenenfalls im Vakuum oder gegebenenfalls durch Waschen mit hydrophilen organischen Flüssigkeiten wie Alkoholen oder Phenolen, oder durch azeotrope Destillation mit organischen Flüssigkeiten wie Phenol, Toluol, Xylol, Methylenchlorid u. a. getrocknet. Danach wird das Ionenaustauscherharz mit dem für die Bisphenolherstellung erforderlichen Phenol gespült und in diesem Medium bei Temperaturen oberhalb des Schmelzpunktes dieses Phenols mit der gewünschten Menge Thiazolin der Formel (I) unter Rühren oder in Wirbelschicht versetzt.

Als Thiazolin der Formel (I) wird vorzugsweise 2-Mercaptothiazolin verwendet.

Das durch Neutralisation modifizierte Kationenaustauscherharz kann zur Herstellung zahlreicher Bisphenole aus Phenolen und Carbonylverbindungen eingesetzt werden.

Geeignete Phenole sind z. B. solche der Formel (II)

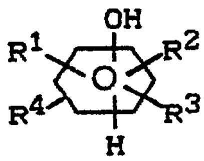 (II),

in welcher

$R^1$, $R^2$, $R^3$ und $R^4$ unabhängig voneinander für Wasserstoff (H), $C_1$-$C_4$-Alkyl oder Halogen wie F, Cl oder Br stehen.

Beispielsweise seien 2.6-Dimethylphenol, o- und m-Kresol, o-sek. Butylphenol, o-tert. Butylphenol, 1.3.5 Xylenol, 2.6-Di-Tert.-butylphenol, Tetramethylphenol, 2-Methyl-6-tert.-butylphenol, o-Phenylphenol, o- und m-Chlorphenol, o-Bromphenol, 6-Chlor-o-Kresol und 2.6-Dichlorphenol genannt.

Bevorzugt ist unsubstituiertes Phenol.

Geeignete Carbonylverbindungen sind solche der Formel (III)

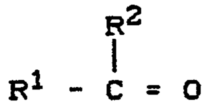 (III),

in welcher

$R^1$ und $R^2$ unabhängig voneinander Wasserstoff (H), $C_1$-$C_6$-Alkyl-, $C_6$-$X_{10}$-Cycloalkyl, $C_6$-$X_{14}$ Aryl, $C_7$-$C_{20}$-Aralkyl, $C_7$-$C_{20}$ Alkylaryl bedeuten oder $R^1$ und $R^2$ gemeinsam einen gesättigten Ring mit 5 bis 6-Ringatomen bilden.

Geeignete Carbonylverbindungen sind z. B. Aldehyde und Ketone, wie Formaldehyd, Methylethylketon, Methylpropylketon, Diethylketon, Cyclohexanon, Acetophenon etc. Bevorzugt ist Aceton.

Die Umsetzung der carbonylverbindung der Formel (III) mit den Phenolen der Formel (II) in Gegenwart der Ionenaustauscher kann kontinuierlich oder diskontinuierlich durchgeführt werden. Die Verfahrensweise und die erforderlichen Apparate sind bekannt.

Bei diskontinuierlicher Durchführung werden pro Mol Carbonylverbindung 80 bis 200 g, vorzugsweise 100 bis 150 g Trockensubstanz an erfindungsgemäßem Ionenaustauscherharz eingesetzt.

Die Reaktionstemperatur bei der Herstellung der Bisphenole liegt im Bereich von 40 - 120°C, vorzugsweise oberhalb des Erstarrungspunktes der beteiligten Komponenten.

Die Reaktions- bzw. Verweilzeit wird so gewählt, daß ein vollständiger Umsatz der verwendeten Carbonylverbindung erzielt wird. Sie beträgt vorzugseise 30 bis 240 Minuten.

Ein weiterer Gegenstand der vorliegenden Erfindung ist somit auch die Verwendung der erfindungsgemäß erhältlichen, mit Thiazolinen der Formel (I) modifizierten, Ionenaustauscher zur Herstellung von Bisphenolen aus Phenolen und Carbonylverbindungen.

Das nach der Umsetzung von Phenol und Carbonylverbindung erhaltene Reaktionsgemisch wird nach üblichen Methoden wie Destillation, Kristallisation etc., aufgearbeitet.

So kann z. B. das hergestellte Bisphenol in der Reaktionsmischung bis zur beginnenden Kristallisation abgekühlt und aus den abfiltrierten Bisphenol-Phenol-Mischkristallen das Phenol durch Destillation oder Extraktion entfernt werden.

Das nach diesem Verfahren hergestellte Bisphenol kann ohne Nachreinigung für bekannte Anwendungsgebiete eingesetzt werden und eignet sich auch für besonders anspruchsvolle Anwendungen wie der Herstellung optisch hochreiner Polycarbonate.

Beispiel 1

Herstellung des modifizierten Ionenaustauscherharzes

Der wasserfeuchte Ionenaustauscher mit ca. 80 Gew.-% Feuchte und einer Totalkapazität von 0,75 mval/ml in Lieferform wird zunächst mit destilliertem Wasser gewaschen. Anschließend wird das Harz bei 90 - 100°C und Wasserstrahlpumpenvakuum 24 h lang getrocknet, so daß der Wassergehalt auf <1 Gew.-% fällt.

Das Restwasser wird im azeotropen Gemische mit Toluol abdestilliert und danach das dem Ionenaustauscherharz noch anhaftende Toluol bei 95°C im Wasserstrahlpumpenvakuum abdestilliert.

120 g des so vorbehandelten Ionenaustauscherharzes werden in einer Rührapparatur in 1128 g Phenol aufgenommen und 24 h unter Feuchtigkeitsauschluß bei 65°C gequollen. Danach wird unter Rühren die für eine bestimmte Belegung in mol-% der Beispiele 2 - 10 erforderliche Menge 2-Mercaptothiazolin zugegeben.

Beispiele 2 - 10

Herstellung von Bisphenol A (BPA)

Mit dem unter Beispiel 1 beschriebenen Verfahren wurde das Ionenaustauscherharz mit 2-Mercaptothiazolin so modifiziert, daß in neun Einstellungen 15-100 % der Sulfonsäuregruppen neutralisiert wurden. Zu der in Beispiel 1 bereiteten Lösung wurden jeweils 58 g Aceton bei 65°C gegeben und nach vollständigem Acetonumsatz die gaschromatographische Reinheit des Bisphenols bzw. die Menge der Nebenkomponenten bestimmt. Die folgende Tabelle faßt die Ergebnisse in Form der Mittelwerte aus je 5 Versuchen/-Beispiel zusammen.

| | | GC-Flächenprozente | |
|---|---|---|---|
| Beispiel | Belegung | BPA | Nebenprodukte |
| 2 | 15 | 93,1 | 6,9 (Vergleich) |
| 3 | 20 | 93,5 | 6,5 (Vergleich) |
| 4 | 25 | 93,7 | 6,3 (Vergleich) |
| 5 | 30 | 94,1 | 5,9 |
| 6 | 40 | 94,6 | 5,4 |
| 7 | 50 | 95,1 | 4,9 |
| 8 | 60 | 95,7 | 4,3 |
| 9 | 80 | 96,7 | 3,3 |
| 10 | 100 | 97,2 | 2,8 |

## Patentansprüche

1. Mit Thiazolinen modifizierter Ionenaustauscher, dadurch gekennzeichnet, daß man bekannte saure Ionenaustauscherharze, mit Totalkapazitäten an Säurefunktionen in wasserfeuchter Form mit einem Wassergehalt von ca. 75 bis 85 Gew.-%, von 0,7 bis 2,1 mval/ml Ionenaustauscher oder mit Totalkapazitäten an Säurefunktionen von 3,5 bis 5 mval, bezogen auf 1 g Trockensubstanz, an Ionenaustauscher, trocknet, danach mit dem für die Bisphenolherstellung einzusetzenden Phenol spült und danach oberhalb des Schmelzpunktes dieses Phenols mit mindestens 0,3 Molen, pro Mol Säurefunktion der Ionenaustauscher, an Thiazolinen der Formel (I)

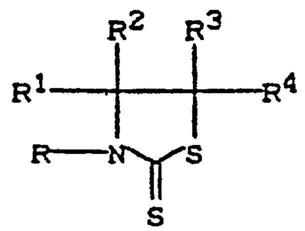

(I),

in welcher
die Reste R, R1, R2, R3, R4 unabhängig voneinander für Wasserstoff, einen $C_1$–$C_6$-Alkyl-, einen $C_5$–$C_{10}$ Cycloalkyl- oder einen $C_6$–$C_{14}$-Arylrest stehen, neutralisiert.

2. Ionenaustauscher gemäß Anspruch 1, dadurch gekennzeichnet, daß man mit 0,4 Molen bis 1 Mol an Thiazolinen der Formel (I) neutralisiert.

3. Verwendung von Ionenaustauschern nach Anspruch 1 zur Herstellung von Kondensationsprodukten.

## Claims

1. Thiazoline-modified ion exchangers, characterized in that known acidic ion exchanger resins having total capacities of acid functions in water-moist form, with a water content of approximately 75 to 85% by weight, in the range from 0.7 to 2.1 mval/ml ion exchanger or having total capacities of acid functions in the range from 3.5 to 5 mval, based on 1 g dry matter, of ion exchanger are dried, subsequently washed with the phenol to be used for bisphenol production and then neutralized with at least 0.3 mol – per mol acid function of the ion exchanger – of thiazolines corresponding to formula (I)

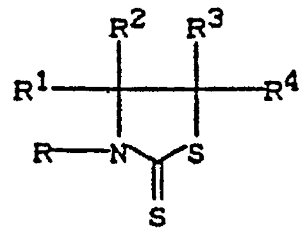

(I),

in which R, $R^1$, $R^2$, $R^3$ and $R^4$ independently of one another represent hydrogen, a $C_{1-6}$ alkyl, a $C_{5-10}$ cycloalkyl or a $C_{6-14}$ aryl radical, at a temperature above the melting point of that phenol.

2. Ion exchangers as claimed in claim 1, characterized in that 0.4 mol to 1 mol thiazolines corresponding to formula (I) are used for neutralization.

3. The use of the ion exchangers claimed in claim 1 for the production of condensation products.

## Revendications

1. Echangeur d'ions modifié par des thiazolines, caractérisé en ce que l'on sèche des résines échangeuses d'ions acides connues ayant des capacités totales en fonctions acides sous forme imprégnée d'eau à une teneur en eau d'environ 75 à 85% en poids de 0,7 à 2,1 mval/ml d'échangeur d'ions ou des capacités totales en fonctions acides de 3,5 à 5 mval, rapportées à 1 g de substance sèche de l'échangeur d'ions, ensuite on les rince avec le phénol à utiliser pour la fabrication du bisphénol et ensuite on les neutralise au-dessus du point de fusion de ce phénol avec au moins 0,3 mol par mol de fonctions acides de l'échangeur d'ions de thiazolines de formule (I)

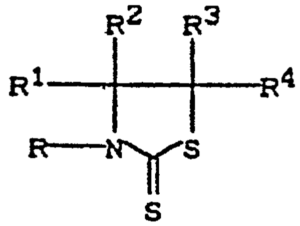

(I),

dans laquelle les restes R, $R^1$, $R^2$, $R^3$ et $R^4$ représentent indépendamment les uns des autres l'hydrogène, un reste alkyle en $C_1$–$C_6$, un reste cycloalkyle en $C_5$–$C_{10}$ ou un reste aryle en $C_6$–$C_{14}$.

2. Echangeur d'ions selon la revendication 1, caractérisé en ce que l'on neutralise par 0,4 à 1 mol de thiazolines de formule (I).

3. Utilisation d'échangeurs d'ions selon la revendication 1 pour la fabrication de produits de condensation.